# EUROPEAN PATENT SPECIFICATION

(11) **EP 2 352 813 B1**
(45) Date of publication and mention of the grant of the patent: **03.10.2012**
(21) Application number: 09745233.8
(22) Date of filing: 29.10.2009
(51) Int. Cl.: C12M 3/08

(54) **APPARATUS AND METHODS FOR PROCESSING TISSUE TO RELEASE CELLS**
VORRICHTUNG UND VERFAHREN FÜR DIE AUFARBEITUNG VON GEWEBE ZUR FREISETZUNG VON ZELLEN
APPAREIL ET PROCÉDÉS POUR TRAITER UN TISSU POUR LIBÉRER DES CELLULES

(30) Priority: 03.11.2008 US 263984; 20.03.2009 US 407946
(43) Date of publication of application: 10.08.2011
(73) Proprietor: Baxter International Inc., Deerfield, Illinois 60015-4633 (US); Baxter Healthcare SA, 8152 Glattpark (Opfikon) (CH)
(72) Inventor: MIN, Kyungyoon, Kildeer, IL 60047 (US)
(74) Representative: Dee, Ian Mark
(86) International application number: PCT/US2009/062584
(87) International publication number: WO 2010/062665

(56) References cited:
- EP-A- 0 512 769
- EP-A- 0 984 060
- WO-A-99/02959
- WO-A-2008/092697
- DE-U1-202004 010 672

## Description

### Technical Field

The present subject matter generally relates to an apparatus and methods for processing tissue to obtain cells.

### Background

Biological material often is used in therapeutic, diagnostic or research applications. However, it may be preferable that the material be separated from the tissue from which it derives before being used in these applications. For example, stem cells may originate from several types of tissue including adipose tissue, muscle and blood. It may be desirable to separate the stem cells from the tissue(s) before further processing for introduction into patients or for use in other applications.

To separate biological material from tissue, the tissue often is subjected to a disaggregation or disassociation process. The tissue disaggregation process may involve mechanical means such as homogenization and sonication. In many instances, it may also involve the use of reagents such as enzymes that digest, dissolve or alter the structure of the tissue to effect release of a desired material. For example, to obtain stem cells from an adipose tissue, a solution of an enzyme such as collagenase may be added to digest the connective tissue component of the adipose tissue, thereby releasing the desired stem cells. The use of enzymes such as collagenase may require the control of temperature, pH and other variables during the tissue disaggregation process.

After or even during disaggregation of tissue, the desired material may be subjected to various purification steps, possibly including filtration, centrifugation and affinity methods. The documents EP 0984060 A2 and EP 0512769 A2 disclose devices and methods for tissue disaggregation and isolation of fat or endothelial cells. There remains a need for an apparatus and methods for processing tissue, including disaggregating and purifying steps, to obtain biological material, including cells.

### Summary

In one aspect, the present invention is directed to an apparatus for processing tissue to release cells from the tissue. The apparatus includes a first housing having an outer wall that has a selected shape. The first housing is adapted to receive a tissue sample. The outer wall of the first housing is sufficiently porous to allow passage therethrough of material including cells derived from the tissue. The apparatus also includes a second housing that at least substantially encloses the first housing and has an outer wall having a selected shape and being spaced apart from the outer wall of the first housing so as to define a gap therebetween, the gap between the outer wall of the first housing and the outer wall of the second housing having either a uniform width or varying continuously in width. At least one of the first and second housings is movable relative to the other of the first and second housings to assist in processing of tissue in the first housing and passage of material including cells derived from the tissue through the porous outer wall of the first housing.

In an embodiment, the outer wall has a selected shape that is substantially the same shape as the selected shape of the outer wall of the first housing or that varies continuously relative to the selected shape of the outer wall of the first housing. The first and second housings further are disposed at an angle of less than 90° relative to a horizontal plane and the first housing is movable relative to the second housing to assist in moving a fluid over the tissue in the first housing.

The disclosure also is directed to methods for processing tissue. In one example, tissue processing may include releasing cells from tissue. In this example, a tissue sample containing cells is inserted into a first housing. The first housing has an outer wall having a selected shape and being sufficiently porous to allow passage therethrough of material including cells derived from the tissue sample. The first housing is at least substantially enclosed by a second housing having an outer wall that has a selected shape that is substantially similar to the selected shape of the outer wall of the first housing or that varies continuously relative to the selected shape of the outer wall of the first housing. The processing further includes introducing tissue-releasing agents into at least one of the housings. The processing also includes moving at least one of the first and second housings relative to the other of the first and second housings to process the tissue sample and to pass material including cells derived from the tissue sample through the porous outer wall of the first housing.

### Brief Description Of The Drawings

Fig. 1 is a cross-sectional diagrammatic view of an example apparatus for processing tissue;
Fig. 2a is a perspective view of an example apparatus for processing tissue;
Fig. 2b is a partial cross-sectional perspective view of the example shown in Fig. 2a;
Fig. 2c is an exploded view of the apparatus of Fig. 2a:
Fig. 3a is a perspective view of another example apparatus for processing tissue;
Fig. 3b is a partial cross-sectional perspective view of the example shown in Fig. 3a;
Fig. 3c is an exploded view of the apparatus shown in Fig. 3a;
Fig. 4a is a perspective view of a further example of an apparatus for processing tissue;
Fig. 4b is an exploded view of the apparatus shown in Fig. 4a;
Fig. 5 is a partial cross-sectional view of the apparatus for processing tissue of Fig. 4a;
Fig. 6 is a schematic flow chart of exemplary steps for processing tissue.
Fig. 7 is a cross-sectional diagrammatic view of a further example of an apparatus for processing tissue employing an agitator.

### Detailed Description

While detailed examples are disclosed herein, it is to be understood that these disclosed examples are merely exemplary, and various aspects and features described herein may have utility alone or in combination with other features or aspects in a manner other than explicitly shown but would be apparent to a person of ordinary skill in the art.

The subject matter of this application is directed generally to an apparatus and method for processing tissue to obtain biological material. In a preferred example, the apparatus is used to process adipose tissue to release cells, particularly stem cells.

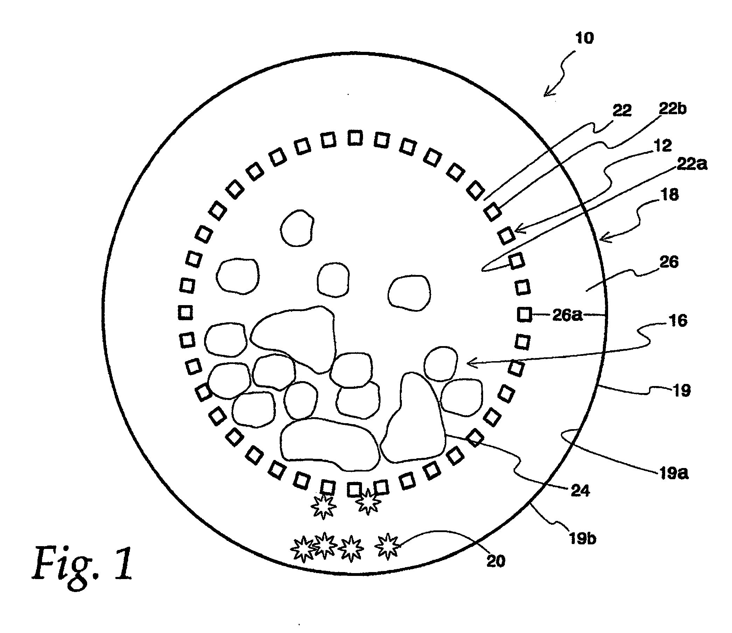

In accordance with this description, an apparatus for processing tissue is shown in a cross-sectional diagrammatic view generally at 10 in Fig. 1. The apparatus includes a first housing 12 having an outer wall 22. The outer wall has an inner surface 22a and an outer surface 22b. The first housing 12 is adapted to receive a tissue sample 16. The apparatus 10 also includes a second housing 18, sized such that the first housing 12 is substantially located within or enclosed by the second housing 18. The second housing 18 includes an outer wall 19 having an inner surface 19a and an outer surface 19b.

At least a portion of the outer wall 22 of the first housing 12 is porous. The porous portion of the outer wall 22 of the first housing 12 allows desired material to pass therethrough while other, undesired material is retained in the first housing. For example, cells 20 may be released from the tissue sample 16 during a disaggregation procedure and may pass from inside the first housing 12 through the pores of the outer wall 22 of the first housing 12, while larger tissue fragments 24 may be retained in the first housing 12. The cells 20 that pass through the porous portion of the outer wall of the first housing 12 may pass into a space or gap 26 between the first and second housings 12, 18.

In one example, the outer walls 22, 19 of the first and second housings 12, 18 have substantially the same shape. More specifically, the inner surface 19a of the outer wall 19 of the second housing 18 (in the illustrated embodiment) is substantially cylindrical and the outer surface 22b of the outer wall 22 of the first housing 12 also is substantially cylindrical, defining a gap 26 therebetween. The first and second housings may be coaxial, thereby defining a gap of substantially uniform width 26a between them. Alternatively, if desired, the respective axes may be offset to define a gap of varying width. Also, although the illustrated assembly employs housings that are both cylindrical, it is not necessarily required that either or both be of cylindrical shape.

The outer and inner diameters, respectively, of the walls 22, 19 of the first and second housings 12, 18 are selected such that the inner surface 19a of the second housing 18 circumscribes the outer surface 22b of the first housing 12. In other words, the inner and outer surfaces 19a and 22b are in a facing arrangement and define the gap 26 therebetween, i.e., between the outer walls of the first and second housings. The width of the gap may be selected for the desired separation, the amount of dissociation fluid to be used, and to create or limit, as desired, shear forces or turbulence within the gap. For instance, in one example in Fig. 1, although not drawn to scale, the outer wall 22 of the first housing 12 may be cylindrical, as shown, and have an outer diameter of from about 10 cm to about 12 cm. The outer wall 19 of the second housing 18, in turn, may be cylindrical and have an inner diameter of from about 12 cm to about 15 cm. Also for example, the gap 26 between the outer surface 22b of wall 22 of the first housing 12 and the inner surface 19a of wall 19 of the second housing 18 may have a gap width 26a in the range of from about 1 cm to about 2.5 cm. This may correspond to the example apparatus having a capacity for receipt of about 500 ml of tissue within the first housing 12 and a total capacity of about 700 ml within the second housing 18.

As noted above, the first and second housings 12, 18 preferably share a common longitudinal axis, and with appropriate corresponding substantially similar shapes, a substantially uniform gap 26 may be provided between the outer surface 22b of the outer wall 22 of the first housing 12 and the inner surface 19a of the outer wall 19 of the second housing 18. However, it will be appreciated that the housings alternatively may not share a common axis, in which event the gap width 26a would not be uniform, but would vary continuously around the device. In addition, for preselected lengths of the housings, a smaller gap 26 will result in a smaller spacing having a smaller relative volume. Accordingly, a smaller gap width 26a between the outer surface 22b of the outer wall 22 of the first housing 12 and the inner surface 19a of the outer wall 19 of the second housing 18 will require a smaller volume of the potentially expensive solutions used for processing the tissue to completely immerse the tissue sample, including for disaggregation of the tissue. Thus, smaller amounts of disaggregation reagents such as enzymes may be required. This may provide a cost benefit.

In addition, the gap width and relative rotational speed of the housing(s) may be selected for fluid dynamic/processing reasons. For instance, a smaller gap width 26a may, for a given diameter or relative rotational speed between the housings 12 and 18, increase the shear forces or induce turbulence, for example vortices within the gap, to which the fluid is subjected. This may help ensure the more complete mixing of reagents and tissue during processing or enhance the passage of cells through the porous first housing outer wall. Higher shear forces also may be achieved with a higher relative rotational speed, such as, for example, if the first housing were to rotate at a higher speed while the second housing did not rotate, or vice versa, or if both housings were to rotate but in opposite directions so as to yield an increased relative rotational speed difference between the two housings. Similarly, if it is desired to subject more delicate tissues or cells to lower fluid shear forces, a larger gap width 26a may be selected when determining the relative diameters of the outer walls 22,19 of the first and second housings 12, 18, and/or a lower relative rotational speed may be used.

Further, the gap width between the first and second housings may vary in an axial direction. Thus, for example, when working with a lighter tissue, such as adipose tissue, the gap width could be wider or narrower at one end of the apparatus. More specifically, the first housing could have an outer wall with a larger outer diameter at the top of the first housing than at its bottom, forming a truncated conical shape. The outer diameter of the first housing may be, as one example, 0.5 cm to 1.25 cm larger at the top than at the bottom, thus having a shape that varies continuously, while the second housing could have an outer wall with a constant diameter, thus having a cylindrical internal shape. This would create a smaller gap nearer the top of the apparatus and potentially create higher shear forces in such location to assist in the processing of the lighter adipose cells that may tend to float to or otherwise accumulate in this upper region. Such a smaller gap nearer the top of the apparatus also may be achieved with a first housing having a cylindrical outer wall and a second housing having an outer wall with a truncated conical shape having a smaller diameter at the top of the second housing than at its bottom.

For processing of some tissues, it may be desirable, at a given rotational speed, to generate higher shear forces nearer to the bottom of the apparatus. In such instances, this could be achieved if, for example, the first housing has an outer wall with a truncated conical shape having a smaller outer diameter at the top of the first housing than at its bottom, while the second housing has an outer wall with a cylindrical internal shape with a constant diameter. The larger gap nearer the top of the apparatus potentially would create lower shear forces in such location relative to the higher shear forces generated nearer the bottom of the apparatus. Such a smaller gap nearer the bottom of the apparatus also may be achieved with a first housing having a cylindrical outer wall and a second housing having an outer wall with a varied diameter, such as a truncated conical shape having a larger diameter at the top of the second housing than at its bottom.

Thus, it will be appreciated that both rotational speed and the gap between housings may be selected in the design of apparatus for particular processing procedures to achieve desired shear forces that will prevent plugging of the pores in the outer wall of the first housing and to assist in creating dissociation of the tissue.

When constructing the apparatus, the first and second housings 12, 18 may be formed from one or more of a variety of materials, including disposable materials. In a preferred example, the housings also are formed from materials in a manner to make the housings substantially rigid. The materials may include glass, plastic, and metal, and/or combinations of such materials. In one example, the second housing may be composed, at least in part, of a relatively transparent material that allows the space enclosed by the second housing, including the first housing, to be visualized.

In an example apparatus, the porous portion of the outer wall 22 of the first housing 12 may be formed from a mesh panel. The mesh panel may include a molded sheet having apertures, a non-woven membrane or a web or net structure having strands of one or more materials that are woven together to form a porous structure. Materials useful in this apparatus may be of the type described in U.S. Patent Nos. 6,491,819; 5,194,145; 6,497,821, or in U.S. Published Application No. 20050263452. The materials of the mesh panel may be coated with materials that prevent tissue, cells, molecules or reagents from adhering to or chemically reacting with the outer wall 22. The porous portion, for example, may include metal wire woven together and coated with Teflon. Regardless of their respective shape(s), the pores of the outer wall 22 may be sized so as to be the equivalent of being in a range from about 5µm to about 3000 µm in diameter. In a preferred example, the pores are equivalent to being about 200 µm or larger in diameter. Additionally, the inner surface 22a and outer surface 22b of the outer wall 22 of the first housing 12 may be modified such that tissue processing or purification agents are bound to or incorporated into the outer wall 22 materials.

In various examples, at least one of the first and second housings 12, 18 may be moveable to assist in the processing of tissue and the passage of material such as cells through the porous outer wall 22 of the first housing 12. The housings may be shaken, rotated, agitated or otherwise moved, as desired. The movement of one or both housings may, for example, prevent tissue fragments 24 from adhering to the first housing 12 and may also facilitate the even distribution of the tissue-releasing agent(s) throughout the tissue sample.

In one example, the first housing is rotated relative to the second housing. The rotation speed may be, for example, about one revolution per second. However, it will be appreciated that other speeds may be chosen as desired. Such rotating action may be used to increase the shear rate between the porous outer wall 22 of the first housing 12 and the liquid within the space 26 to prevent plugging of the porous outer wall 22 by the solid portion of the tissue or other materials used in the processing. Thus, the rotating speed can be varied to achieve a desired shear rate at the surface of the porous outer wall 22 of the first housing 12. While continuous rotation of one housing relative to another may be preferred, rotational oscillation or varying the rotational speed and/or direction of one housing relative to the other may be used to increase the rate or degree of dissociation.

In some examples, movement of the housings may be accomplished by fitting the housings into a durable or reusable device with an underlying base which may include devices such as one or more motors which are adapted to interact with and move the housings. The base may also include devices to control and monitor the temperature, pH and other variables.

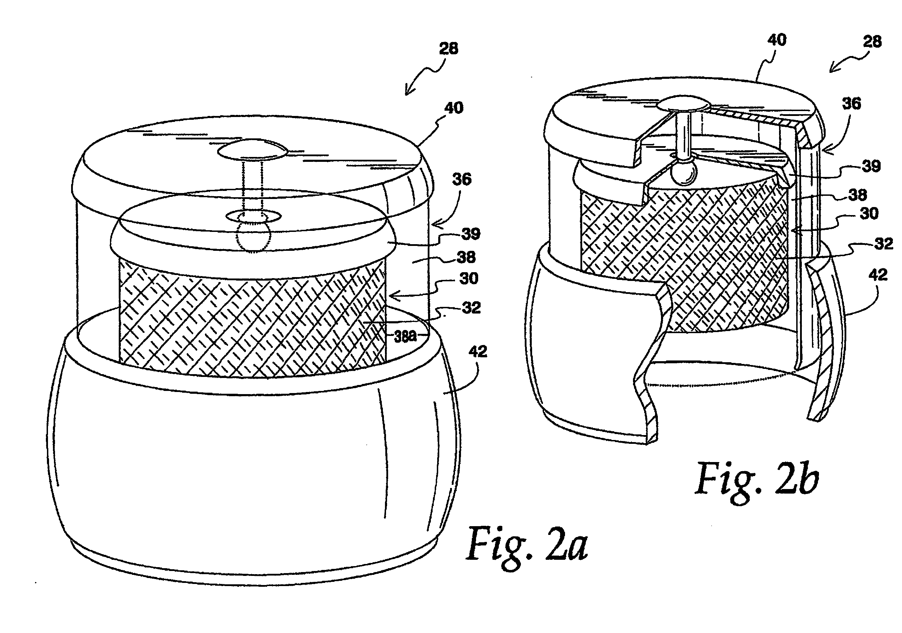

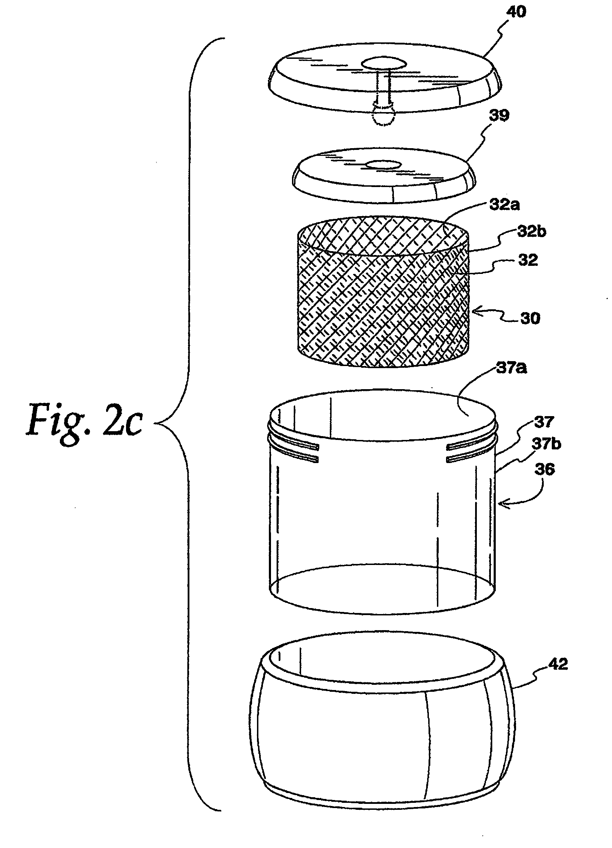

Turning now to Figs, 2a-2c, an example of a tissue processing apparatus is shown in three views. The apparatus 28 includes a first housing 30 that includes a porous outer wall 32 having an inner surface 32a and an outer surface 32b. Although the outer wall 32 of the first housing 30 is shown as being almost entirely porous in this example, the wall may be porous only in part, as desired. Also, in this example, the outer wall 32 of the first housing 30 is substantially cylindrical. The pore size of the wall 32 is selected, such as within the above-disclosed ranges, to allow passage of desired biological material, such as cells derived from the tissue that is placed in the first housing 30. As shown in this example, the first housing 30 is enclosed by a second housing 36. The second housing 36 includes an outer wall 37 having an inner surface 37a and an outer surface 37b. The outer wall 37 also is substantially cylindrical, and therefore of substantially the same shape as the outer wall 32 of the first housing 30. There may be a space or gap 38 defined between the opposed facing outer surface 32b of the first housing wall 32 and the inner surface 37a of the second housing wall 37. The respective diameters of the outer walls 32, 37 of the housings 30, 36, and the relative gap width 38a between the outer surface 32b of the outer wall 32 of the first housing 30 and the inner surface 37a of the outer wall 37 of the second housing 36 preferably fall within the ranges discussed above with respect to the example in Fig. 1, but may be varied as desired for the intended process. In addition, the first and second housings 30, 36 may be removable to facilitate processing, cleaning, or for other purposes. In the example shown in Figs. 2a-2c, the first and second housings 30, 36 may have lids or covers 39, 40, that fit an upper opening of the respective housings. The lids may seal the contents of the apparatus 28 from the external environment. The lids or covers 39, 40 may be removable to facilitate placement or removal of tissue, or to otherwise allow access to the contents of the housings when desired. The bottom of each respective housing also may contain a lid or cover (not shown) or the outer wall of each housing may be extended to form a bottom wall or surface.

As noted above, the first and second housings may be adapted to fit into a base structure 42. The base 42 may contain a motor for shaking, rotating or otherwise moving the first housing 30 relative to the second housing 36 to agitate at least one of the housings and facilitate tissue disaggregation, and the release of cells from a tissue sample. The base structure also may include devices to control and monitor temperature, pH or other suitable variables. The housings and associated base may employ the principles and structures illustrated in U.S. Patent No. 5,194,145 in which relative rotation between inner and outer housings creates shear stress to relieve plugging within the device for enhanced filtration.

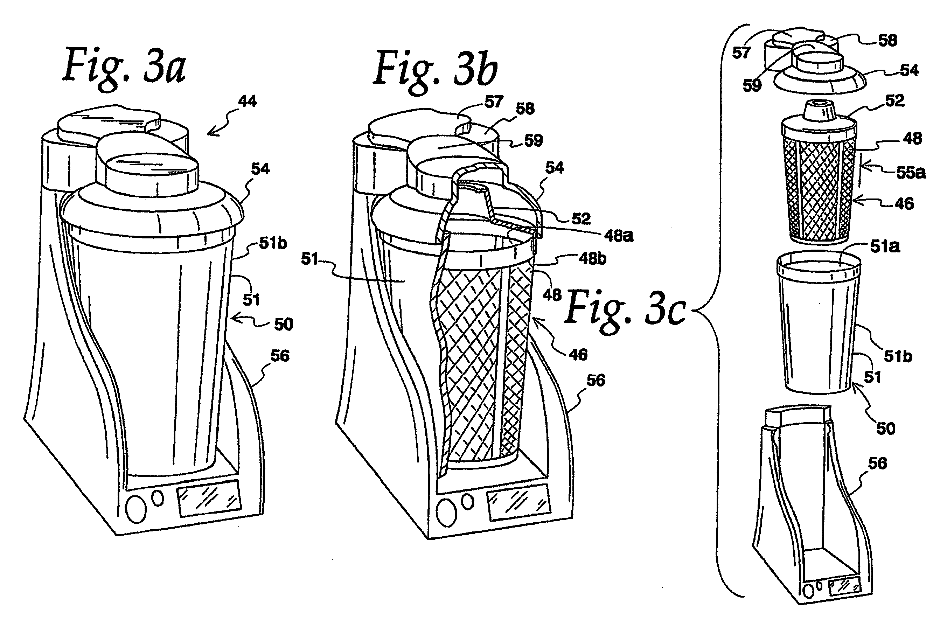

Figs. 3a-3c illustrate a further example of an apparatus 44 according to the disclosure. As with the previous examples, a first housing 46 has an outer wall 48 that is adapted to receive a tissue sample. The outer wall 48 includes an inner surface 48a and an outer surface 48b and, consistent with the above examples, is sufficiently porous to allow passage therethrough of material, including cells, derived from the tissue sample while preferably retaining undesired material. The first housing 46 is enclosed by a second housing 50 having an outer wall 51 that includes an inner surface 51 a and an outer surface 51 b.

The first and second housings may have lids or covers 52, 54, respectively, and similarly shaped outer walls, which in this example are of a truncated conical shape, or as discussed above, may have dissimilar shapes that result in a varied gap between the housings. Thus, the first housing 46 and second housing 50 may be configured so as to form a gap 55 of relatively uniform gap width 55a between the outer surface 48b of the outer wall 48 of the first housing 46 and the inner surface 51a of the outer wall 51 of the second housing 50, if the housing outer walls 48, 51 correspond in size and have substantially the same shape. This gap width 55a may be of a preselected size, resulting in a given space between these surfaces, as discussed above with the previous examples. The gap width 55a between the respective surfaces may be selected depending on factors such as those previously discussed with respect to shear forces, required reagent volumes or other factors, and again may be varied depending on the shape of the respective housings.

A base structure 56 may include devices to rotate the first and/or second housing or agitate at least one of the housings relative to the other and also may include monitors and related systems to detect and control temperature, pH and other variables, as desired. In this example, the base 56 includes a motor, such as a gear or magnetic drive (not shown) which is adapted to drive a cooperative gear or magnetic coupling 57 on a base cover 58, to cause rotation of the first housing 46 within the second housing 50 at a fixed or variable speed. Again, although the first and second housings are illustrated as concentric, the axes may be offset to provide a gap 55 of varying gap width 55a at different circumferential locations around the gap, and the gap width could vary axially if the housings are not of the same shape.

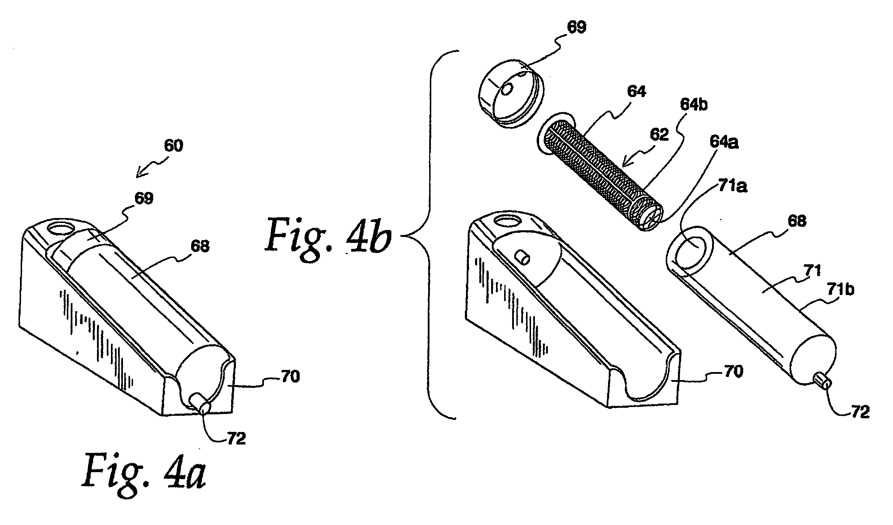

Figs. 4a and 4b show another example of an apparatus 60 according to the disclosure. In this example, a first housing 62 includes an outer wall 64 and is adapted to receive a tissue sample. The outer wall 64 is sufficiently porous to allow passage therethrough of material including cells derived from the tissue, and has an inner surface 64a and an outer surface 64b. The first housing 62 is enclosed by a second housing 68 with a cover 69. The second housing 68 includes an outer wall 71 having an inner surface 71 a and an outer surface 71 b. The housing sizing and gap between the housings is intended to be within the above-disclosed ranges, and it will be appreciated that in this example the respective housing outer walls 64, 71 are substantially of the same cylindrical shape. In this example, the first and second housings 62, 68 are positioned in a base 70 at an angle less than 90° to the surface on which the apparatus 60 rests. This angled or reclined positioning increases the surface area of the tissue within the first housing that may be exposed to a fluid or solution placed in the apparatus 60 if the fluid does not completely fill the second housing 68. In this way, less solution may be used while making contact with more of the tissue in the first housing. As with the selection of the gap and spacing between the outer surface 64b of the outer wall 64 of the first housing 62 and the inner surface 71 a of the outer wall 71 of the second housing 68, this may provide a further manner in which to limit the fluids required to achieve the desired processing.

The base 70 may include a motor that may be used to rotate the first housing 62 relative to the second housing 68 to enhance processing of the tissue sample and passage of material, including cells, through the porous outer wall 64. The base 70 also may include devices to control and monitor temperature, pH and other variables, as desired. In addition, a port 72 may be present in the bottom of the second housing 68 to allow the flow of fluids, including fluids containing biological material such as cells, from the apparatus.

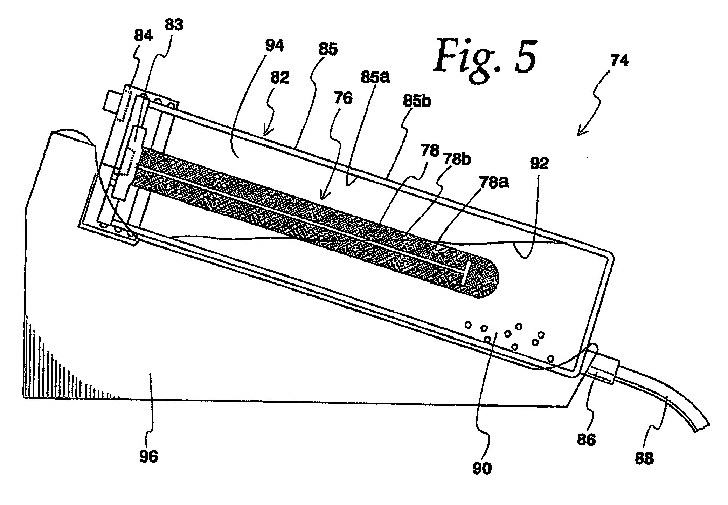

Fig. 5 shows a further example of an apparatus 74 for processing tissue. The cross-sectional view includes released cells 90 and a solution 92, such as a solution of a disaggregation agent. As in previous examples, a first housing 76 includes a porous outer wall 78 and is adapted to receive a tissue sample. While this view again is not to scale, the first housing 76 additionally is shown with a substantially reduced diameter, for description purposes only. The outer wall 78 of the first housing 76 includes an inner surface 78a and an outer surface 78b. The first housing 76 is enclosed by a second housing 82 which includes an outer wall 85 with an inner surface 85a and an outer surface 85b. The first and second housings 76, 82 may have lids or covers 83, 84, respectively, and may be positioned in a base 96. The housings 76, 82 of this example may be sized within the previously disclosed ranges and the outer walls 78, 85 preferably are of corresponding sizes to permit them to be of substantially the same shape, which in this example is illustrated as being cylindrical, although as previously discussed, there may be situations where different cross-sectional shapes and varied gaps between the housings are beneficial.

As in previous examples, the base 96 may include one or more motors or drive units such as magnetically or gear coupled drives that may be used to move at least one of the housings, such as to rotate the first housing relative to the second housing, to enhance processing of tissue in the first housing and passage therethrough of material, including cells, derived from the tissue sample. The base 96 also may include devices to control and monitor temperature, pH and other variables, as desired. In addition, an outlet 86 and tubing 88 may be provided so that the biological material, such as cells 90 released during tissue disaggregation, may be flowed out of the second housing 82 of the tissue processor 74.

In accordance with the description and referring generally to Fig. 5, a method of using an apparatus 74 generally includes inserting a tissue sample containing cells (e.g. adipose tissue containing stem cells) into the first housing 76. The tissue sample is subjected to a disaggregation process while placed in the first housing. The disaggregation process may include adding a solution 92 to facilitate release of biological material. Biological material, such as cells 90, may be released during disaggregation and the cells 90 may flow from the first housing 76, through the porous outer wall 78 of the first housing 76. In this example, cells are shown as initially collecting in the space which is formed largely by the gap 94 of gap width 94a between the outer surface 78b of the outer wall 78 of the first housing 76 and the inner surface 85a of the outer wall 85 of the second housing 82. During the disaggregation procedure, at least one of the first and second housings may be rotated or otherwise agitated relative to the other to facilitate the release of cells from the tissue sample and the flow of the cells through the outer porous outer wall 78 of the first housing 76.

According to this description, the apparatus may be used with numerous tissue sources where disaggregation is desired. For example, the apparatus may be used with adipose tissue or muscle, which are among preferred sources of adult stem cells. The tissue-derived material that may be released includes cells, including individual cells, multi-cellular aggregates and cells associated with non-cellular material. The released cells may include more than one cell type. In some examples, the biological material also may be substantially non-cellular. In a preferred example, the tissue processor may be used to process adipose tissue to release stem cells.

In the example of adipose tissue, tissue may be obtained from a patient using conventional procedures including lipoaspiration or liposuction. The adipose tissue obtained from a patient may then be placed directly into the first housing or initially may be washed or otherwise treated before being placed in the first housing.

In one example, the tissue disaggregation process may involve the enzymatic treatment of the tissue sample. For example, collagenase digestion of connective tissue may be used to affect release of stem cells from adipose tissue. When enzymatic treatment is used, a solution of the enzyme may be added either directly to the first housing 76 where the tissue is located, or added to the space at the gap 94 between the walls of the first and second housings 76, 82 such that the enzyme diffuses from the inter-housing space into the first housing 76.

After or during the disaggregation process, the flow of cells 90 from the first housing 76 through the porous outer wall 78 of the first housing 76 may be facilitated by flowing or pumping cell-compatible fluids through the first housing 76 such that cells are carried from the first housing through the porous outer wall 78 by the flow of the fluids. In one example, there may be a continuous flow of fluid through the first housing 76 to carry cells from the first housing through the porous outer wall 78 and to an outlet 86 located, for example, at the bottom of the second housing 82, as shown for example in Fig. 5.

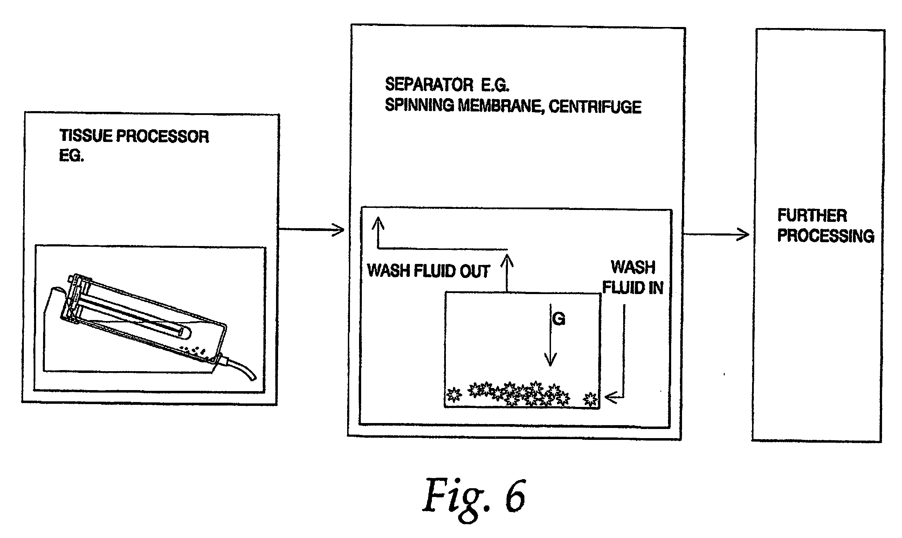

In one example, the apparatus may be directly linked to one or more systems or apparatus for further processing of materials. Tissue-derived material, including cells, may be flowed from the tissue processing apparatus through an outlet and may then flow to systems, such as those that employ a separator, such as a spinning membrane or centrifuge, for washing, reduction in volume, treating, or further processing of the cells, such as for example, purifying via immuno selection, or other suitable processes. Fig. 6 is a schematic flow chart showing how the tissue processing apparatus may be part of larger systems for multi-step treatment and purifying of cells. A pump (not shown), such as a peristaltic or other suitable pump, may be included to facilitate the flow of material, such as cells, from the tissue processing apparatus to cell processing systems.

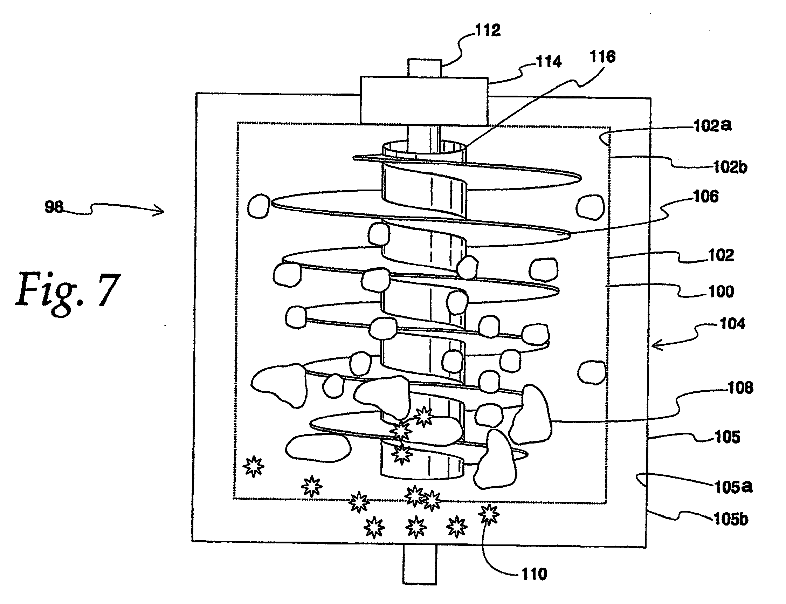

A further example of an apparatus for processing tissue 98 according to the disclosure is shown in Fig. 7. As in previous examples, the apparatus 98 includes a first housing 100 with a porous outer wall 102 having an inner surface 102a and an outer surface 102b. The first housing 102 is adapted to receive a tissue sample and is enclosed within a second housing 104 which includes an outer wall 105 having an inner surface 105a and an outer surface 105b. The relative sizes of the housings 100, 104, and distance between the respective outer walls 102, 105 of this example are in keeping with the ranges previously disclosed. Moreover, the housing outer walls 102, 105 are substantially of the same cylindrical shape.

In this example, the apparatus also includes an agitator 106 that is located within the first housing 100 to enhance tissue processing. The agitator 106 may enhance tissue disaggregation by directly contacting the tissue 108 to disassociate or tear the tissue 108, by creating shear effects within the first housing 100, by improving reagent and tissue mixing or by some combination of these effects.

In the example shown in Fig. 7, the agitator 106 is configured as an auger, although any other suitable configuration, such as a paddle, beater or other implement may be used. The diameter and length of the auger as well as the pitch of the auger flighting may be selected according to particular requirements. An agitator 106, such as an auger, as described here may be used with any of the previously described examples of a tissue processing apparatus.

The apparatus 98 also preferably includes a drive mechanism for moving the agitator 106, e.g. such as rotating the above-disclosed auger. In the example shown in Fig. 7, the auger is driven by a motor 114 via a drive shaft 112 mounted in a bearing 116. It will be appreciated that in other embodiments, it may be desirable to utilize a magnetic drive mechanism to rotate the auger, so that contents of the first and second housings 100, 104 may be completely sealed from the outside environment.

According to this example, as in previous examples, a tissue sample is placed within the first housing 100 which contains an agitator or auger 106. A solution containing a tissue disaggregation agent such as collagenase may be also placed within the first or second housings 100, 104. During processing of the tissue sample, the agitator 106 and the first housing 100 may both rotate relative to the second housing 104. The agitator and first housing 100 may rotate in the same or different directions, continuously or intermittently, and at the same or different speeds relative to each other. In a preferred example, the agitator 106 and the first housing 100 rotate in different directions.

The direct contact of the agitator 106 with the tissue 108 may effect disassociation or tearing of the tissue 108 into smaller fragments, enhancing tissue disaggregation. In addition, rotation of the auger may improve tissue disaggregation due to shear effects on the tissue and improved mixing of tissue and disaggregation reagents. As in previous examples, tissue disaggregation results in larger tissue fragments 108 being retained in the first housing 100 whereas cells 110 pass through the porous outer wall 102 of the first housing 100.

Aspects of the present subject matter described above may be beneficial alone or in combination, with one or more other aspects. Without limiting the foregoing description, in accordance with one aspect of the subject matter herein, there is provided an apparatus for processing tissue to release cells from the tissue where the apparatus comprises a first housing having an outer wall that has a selected shape. The first housing is adapted to receive a tissue sample and the outer wall has an outer surface and is sufficiently porous to allow passage therethrough of material including cells derived from the tissue. The apparatus also comprises a second housing that at least substantially encloses the first housing and has an outer wall that has a selected shape and an inner surface. The inner surface of the outer wall of the second housing is spaced apart from the outer surface of the outer wall of the first housing so as to define a gap therebetween. The gap between the outer surface of the outer wall of the first housing and the inner surface of the outer wall of the second housing has either a uniform width or varies continuously in width. Further, at least one of the first and second housings is movable relative to the other of the first and second housings to assist in the processing of tissue in the first housing and passage of material including cells derived from the tissue through the porous outer wall of the first housing.

In accordance with another aspect which may be used or combined with the preceding aspect, the housings are adapted to fit into a base.

In accordance with another aspect which may be used or combined with the preceding aspect, the base is adapted to move the first housing relative to the second housing.

In accordance with another aspect which may be used or combined with any of the preceding aspect, the base is adapted to rotate the first housing relative to the second housing.

In accordance with another aspect which may be used or combined with any of the preceding three aspects, the base is adapted to hold the first and second housings at an angle of less than 90° relative to a surface on which the base rests during processing.

In accordance with another aspect which may be used or combined with any of the preceding aspects, the second housing outer wall is substantially rigid.

In accordance with another aspect which may be used or combined with any of the preceding aspects, the respective outer walls of said first and second housings are of a substantially cylindrical shape.

In accordance with another aspect which may be used or combined with any of the preceding aspects, the outer wall of the first housing is sufficiently porous to allow passage therethrough of material including cells having a diameter of from about 5 to about 3000 µm.

In accordance with another aspect which may be used or combined with any of the preceding aspects, the wall of the first housing is sufficiently porous to allow passage therethrough of material including cells having a diameter of about 200 µm.

In accordance with another aspect which may be used or combined with any of the preceding aspects, the first housing outer wall further comprises a mesh panel.

In accordance with another aspect which may be used or combined with any of the preceding aspects, the first housing is removable from the second housing.

In accordance with another aspect which may be used or combined with any of the preceding aspects, the apparatus is adapted to be linked to a system for processing cells.

In accordance with another aspect which may be used or combined with any of the preceding aspects, the housings are adapted to receive a tissue-releasing agent in communication with the tissue sample.

In accordance with another aspect which may be used or combined with the preceding aspect, the tissue-releasing agent is an enzyme.

In accordance with another aspect which may be used or combined with any of the preceding aspects, the tissue sample is adipose tissue.

In accordance with another aspect which may be used or combined with any of the preceding aspects, the material including cells comprises stem cells.

In accordance with another aspect which may be used or combined with the preceding aspect, an agitator is movably disposed within the first housing.

In accordance with another aspect which may be used or combined with any of the preceding aspects, the agitator comprises an auger rotatable relative to the first housing.

In accordance with another aspect, there is provided an apparatus for processing tissue to release cells from the tissue, comprising a first housing having an outer wall that has a selected shape. Further, the first housing is adapted to receive a tissue sample and the outer wall is sufficiently porous to allow passage therethrough of material including cells derived from the tissue. The apparatus further includes a second housing that at least substantially encloses the first housing and has an outer wall that has a selected shape that is substantially the same shape as the selected shape of the outer wall of the first housing or that varies continuously relative to the selected shape of the outer wall of the first housing. The first and second housings are disposed at an angle of less than 90° relative to a horizontal plane. The first housing is movable relative to the second housing to assist in moving a fluid over the tissue in the first housing and passing material including cells derived from the tissue through the porous outer wall of the first housing.

In accordance with another aspect which may be used or combined with the preceding aspect, the housings are adapted to fit into a base.

In accordance with another aspect which may be used or combined with the preceding aspect, the base is adapted to move the first housing relative to the second housing.

In accordance with another aspect which may be used or combined with any of the preceding two aspects, the base is adapted to rotate the first housing relative to the second housing.

In accordance with another aspect which may be used or combined with any of the preceding four aspects, the second housing outer wall is substantially rigid.

In accordance with another aspect which may be used or combined with any of the preceding five aspects, the respective outer walls of the first and second housings are of a substantially cylindrical shape.

In accordance with another aspect which may be used or combined with any of the preceding six aspects, the first housing outer wall includes a mesh panel.

In accordance with another aspect which may be used or combined with any of the preceding seven aspects, the first housing is removable from the second housing.

In accordance with another aspect which may be used or combined with any of the preceding eight aspects, the apparatus is adapted to be linked to a system for processing cells.

In accordance with another aspect which may be used or combined with any of the preceding nine aspects, the fluid includes a tissue-releasing agent.

In accordance with another aspect which may be used or combined with the preceding aspect, the tissue-releasing agent is an enzyme.

In accordance with another aspect which may be used or combined with any of the preceding eleven aspects, the tissue sample is adipose tissue.

In accordance with another aspect which may be used or combined with any of the preceding twelve aspects, the material including cells comprises cells.

In accordance with another aspect which may be used or combined with any of the preceding thirteen aspects, an agitator is movably disposed within the first housing.

In accordance with another aspect which may be used or combined with the preceding aspect, the agitator comprises an auger rotatable relative to the first housing.

In accordance with another aspect which may be used or combined with any of the preceding fifteen aspects, the outer wall of the first housing has an outer surface and the outer wall of the second housing has an inner surface and the outer surface of the outer wall of the first housing is spaced apart from inner surface of the outer wall of the second housing to define a gap.

In accordance with another aspect which may be used or combined with any of the preceding sixteen aspects, a gap between the outer surface of the outer wall of the first housing and inner surface of the outer wall of the second housing has an uniform width or varies continuously.

In accordance with another aspect, there is provided a method for processing tissue to release cells from the tissue comprising inserting a tissue sample containing cells into a first housing wherein the first housing has an outer wall having a selected shape and is sufficiently porous to allow passage therethrough of material including cells derived from the tissue sample. Further, the first housing is at least substantially enclosed by a second housing having an outer wall having a selected shape that is substantially similar to the selected shape of the outer wall of the first housing or that varies continuously relative to the selected shape of the outer wall of the first housing. Further, tissue-releasing agents may be introduced into at least one of the housings. At least one of the first and the second housings may be moved relative to the other of the first and second housings to process the tissue sample and to pass material including cells derived from the tissue sample through the porous outer wall of the first housing.

In accordance with another aspect which may be used or combined with preceding aspect, the tissue-releasing agent is added to the first housing.

In accordance with another aspect which may be used or combined with any of the preceding two aspects, the tissue-releasing agent is added to the second housing.

In accordance with another aspect which may be used or combined with any of the preceding three aspects, the tissue-releasing agent is collagenase.

In accordance with another aspect which may be used or combined with any of the preceding four aspects, the tissue sample is adipose tissue.

In accordance with another aspect which may be used or combined with any of the preceding five aspects, the cells derived from the tissue sample comprise stem cells.

In accordance with another aspect which may be used or combined with any of the preceding six aspects, the cells derived from the tissue sample are flowed out of the second housing to a collection device.

In accordance with another aspect which may be used or combined with any of the preceding seven aspects, a pump is used to flow the cells derived from the tissue sample out of the second housing to a collection device.

In accordance with another aspect which may be used or combined with any of the preceding eight aspects, the cells derived from the tissue sample are flowed to an apparatus for further processing.

In accordance with another aspect which may be used or combined with any of the preceding nine aspects, moving at least one of the first and second housings includes moving the first housing relative to the second housing.

In accordance with another aspect which may be used or combined with the preceding aspect, moving the first housing relative to the second housing includes rotating the first housing relative to the second housing.

In accordance with another aspect which may be used or combined with any of the preceding two aspects, moving the first housing relative to the second housing includes shaking the first housing relative to the second housing.

In accordance with another aspect which may be used or combined with any of the preceding twelve aspects, an agitator is placed within the first housing.

In accordance with another aspect which may be used or combined with the preceding aspect, the agitator rotates in one direction and the first housing rotates in an opposite direction.

In accordance with another aspect which may be used or combined with any of the preceding fourteen aspects, the respective outer walls of said first and second housings are of a substantially cylindrical shape.

In accordance with another aspect which may be used or combined with any of the preceding fifteen aspects, the outer wall of the first housing has an outer surface and the outer wall of the second housing has an inner surface and the outer surface of the outer wall of the first housing is spaced apart from inner surface of the outer wall of the second housing to define a gap.

In accordance with another aspect which may be used or combined with any of the preceding sixteen aspects, a gap between the outer surface of the outer wall of the first housing and inner surface of the outer wall of the second housing has a uniform width or varies continuously.

It will be understood that the examples of the present disclosure are illustrative of some of the applications of the principles of the present disclosure.

## Claims

1. An apparatus (10, 28, 44, 60, 98) for processing tissue to release cells from the tissue, comprising:
a first housing (12, 30, 46, 62, 100) having an outer wall (22, 32, 48, 64, 102) that has a selected shape, the first housing adapted to receive a tissue sample and the outer wall having an outer surface (22b, 32b, 48b, 64b, 102b) and being sufficiently porous to allow passage therethrough of material including cells derived from the tissue;
a second housing (18, 36, 50, 68, 104) that at least substantially encloses the first housing and has an outer wall (19, 37, 51, 71, 105) having a selected shape and an inner surface (19a, 37a, 51a, 71a, 105a) being spaced apart from the outer surface of the outer wall of the first housing so as to define a gap (26, 38, 55) therebetween, the gap between the outer surface of the outer wall of the first housing and the inner surface of the outer wall of the second housing having either a uniform width or varying continuously in width; and
at least one of the first and second housings being movable relative to the other of the first and second housings to assist in processing of tissue in the first housing and passage of material including cells derived from the tissue through the porous outer wall of the first housing.

2. The apparatus of claim 1, wherein the housings (30, 36, 46, 50, 62, 68) are adapted to fit into a base (42, 56, 70).

3. The apparatus of claim 2, wherein the base (42, 56, 70) is adapted to move the first housing (30, 46, 62) relative to the second housing (36, 50, 68).

4. The apparatus of claim 3, wherein the base (42, 56, 70) is adapted to rotate the first housing (30, 46, 62) relative to the second housing (36, 50, 68).

5. The apparatus of claim 2, wherein the base (70) is adapted to hold the first and second housings (62, 68) at an angle of less than 90° relative to a surface on which the base rests during processing.

6. The apparatus of claim 1, wherein the second housing outer wall (19, 37, 51, 71) is substantially rigid.

7. The apparatus of claim 1, wherein the outer wall (22, 32, 48, 64) of the first housing (12, 30, 46, 62) is sufficiently porous to allow passage therethrough of material including cells having a diameter of from about 5 to about 3000 µm.

8. The apparatus of claim 1, wherein the first housing outer wall (22, 32, 48, 64) further comprises a mesh panel.

9. The apparatus of claim 1, wherein the housings (12, 30, 46, 62, 18, 36, 50, 68) are adapted to receive a tissue-releasing agent in communication with the tissue sample.

10. The apparatus of claim 1 further comprising an agitator (106) movably disposed within the first housing (100).

11. A method for processing tissue to release cells from the tissue, comprising:
a inserting a tissue sample containing cells into a first housing (12, 30, 46, 62, 100) wherein the first housing has an outer wall (22, 32, 48, 64, 102) having a selected shape and being sufficiently porous to allow passage therethrough of material including cells derived from the tissue sample and wherein the first housing is at least substantially enclosed by a second housing (18, 36, 50, 68, 104) having an outer wall (19, 37, 51, 71, 105) having a selected shape that is substantially similar to the selected shape of the outer wall of the first housing or that varies continuously relative to the selected shape of the outer wall of the first housing;
b introducing tissue-releasing agents into at least one of the housings; and
c moving at least one of the first and the second housings relative to the other of the first and second housings to process the tissue sample and to pass material including cells derived from the tissue sample through the porous outer wall of the first housing.

12. The method of claim 11, wherein the tissue-releasing agent is added to the first housing (12, 30, 46, 62, 100).

13. The method of claim 11, wherein the tissue-releasing agent is added to the second housing (18, 36, 50, 68, 104).

14. The method of claim 11 further comprising flowing the cells derived from the tissue sample to an apparatus for further processing.

15. The method of claim 11, wherein moving at least one of the first and second housings further comprises moving the first housing (12, 30, 46, 62) relative to the second housing (18, 36, 50, 68).

16. The method of claim 11, wherein the tissue sample is adipose tissue.

17. The method of claim 11, wherein the material including cells comprises stem cells.

18. The method of claim 11, wherein the tissue releasing agent is collegenase.

## Patentansprüche

1. Vorrichtung (10, 28, 44, 60, 98) für die Verarbeitung von Gewebe zur Freisetzung von Zellen aus dem Gewebe, umfassend:
Ein erstes Gehäuse(12, 30, 46, 62, 100), das eine äußere Wand (22, 32, 48, 64, 102) aufweist, die eine gewählte Form aufweist, wobei das erste Gehäuse geeignet ist, eine Gewebeprobe aufzunehmen und die äußere Wand eine äußere Fläche (22b, 32b, 48b, 64b, 102b) aufweist und ausreichend porös ist, um den Durchtritt von Material, enthaltend Zellen, die aus dem Gewebe stammen, dadurch zu ermöglichen,
ein zweites Gehäuse (18, 36, 50, 68, 104), welches das erste Gehäuse zumindest im wesentlichen einschließt und eine äußere Wand (19, 37, 51, 71, 105) aufweist, die eine gewählte Form und eine innere Fläche (19a, 37a, 51a, 71a, 105a) aufweist, welche mit einem Abstand von der äußeren Fläche der äußeren Wand des ersten Gehäuses angeordnet ist, so daß ein Spalt (26, 38, 55) dazwischen definiert wird, wobei der Spalt zwischen der äußeren Fläche der äußeren Wand des ersten Gehäuses und der inneren Fläche der äußeren Wand des zweiten Gehäuses entweder eine gleichförmige Breite oder eine sich kontinuierlich ändernde Breite aufweist, und
wobei mindestens eines aus dem ersten und zweiten Gehäuse relativ zu dem anderen aus dem ersten und zweiten Gehäuse beweglich ist, um die Verarbeitung von Gewebe in dem ersten Gehäuse und den Durchtritt von Material, enthaltend Zellen, die aus dem Gewebe stammen, durch die poröse äußere Wand des ersten Gehäuses zu unterstützen.

2. Vorrichtung nach Anspruch 1, wobei die Gehäuse (30, 36, 46, 50, 62, 68) geeignet sind, in eine Basis (42, 56, 70) zu passen.

3. Vorrichtung nach Anspruch 2, wobei die Basis (42, 56, 70) geeignet ist, das erste Gehäuse (30, 46, 62) relativ zu dem zweiten Gehäuse (36, 50, 68) zu bewegen.

4. Vorrichtung nach Anspruch 3, wobei die Basis (42, 56, 70) geeignet ist, das erste Gehäuse (30, 46, 62) relativ zu dem zweiten Gehäuse (36, 50, 68) zu drehen.

5. Vorrichtung nach Anspruch 2, wobei die Basis (70) geeignet ist, das erste und zweite Gehäuse (62, 68) bei einem Winkel von weniger als 90° relativ zu einer Fläche, auf der die Basis während der Verarbeitung ruht, zu halten.

6. Vorrichtung nach Anspruch 1, wobei die äußere Wand des zweiten Gehäuses (19, 37, 51, 71) im wesentlichen starr ist.

7. Vorrichtung nach Anspruch 1, wobei die äußere Wand (22, 32, 48, 64) des ersten Gehäuses (12, 30, 46, 62) ausreichend porös ist, um den Durchtritt von Material, enthaltend Zellen, die einen Durchmesser von etwa 5 bis etwa 3000 µm aufweisen, dadurch zu ermöglichen.

8. Vorrichtung nach Anspruch 1, wobei die äußere Wand des ersten Gehäuses (22, 32, 48, 64) weiter eine Gitterplatte umfaßt.

9. Vorrichtung nach Anspruch 1, wobei die Gehäuse (12, 30, 46, 62, 18, 36, 50, 68) geeignet sind, ein Gewebe-freisetzendes Mittel in Verbindung mit der Gewebeprobe aufzunehmen.

10. Vorrichtung nach Anspruch 1, weiter umfassend ein Rührwerk (106), das beweglich innerhalb des ersten Gehäuses (100) angeordnet ist.

11. Verfahren für die Verarbeitung von Gewebe zur Freisetzung von Zellen aus dem Gewebe, umfassend:
a) Einsetzen einer Gewebeprobe, die Zellen enthält, in ein erstes Gehäuse (12, 30, 46, 62, 100), wobei das erste Gehäuse eine äußere Wand (22, 32, 48, 64, 102) aufweist, die eine gewählte Form aufweist und ausreichend porös ist, um den Durchtritt von Material, enthaltend Zellen, die aus der Gewebeprobe stammen, dadurch zu ermöglichen, und wobei das erste Gehäuse zumindest im wesentlichen von einem zweiten Gehäuse (18, 36, 50, 68, 104) eingeschlossen ist, das eine äußere Wand (19, 37, 51, 71, 105) aufweist, die eine gewählte Form aufweist die im wesentlichen ähnlich zu der gewählten Form der äußeren Wand des ersten Gehäuses ist, oder die sich kontinuierlich relativ zu der gewählten Form der äußeren Wand des ersten Gehäuses ändert,
b) Einbringen Gewebe-freisetzender Mittel in mindestens eines der Gehäuse, und
c) Bewegen mindestens eines aus dem ersten und zweiten Gehäuse relativ zu dem anderen aus dem ersten und zweiten Gehäuse, um die Gewebeprobe zu verarbeiten und Material, enthaltend Zellen, die aus der Gewebeprobe stammen, durch die poröse äußere Wand des ersten Gehäuses durchtreten zu lassen.

12. Verfahren nach Anspruch 11, wobei das Gewebe-freisetzende Mittel in das erste Gehäuse (12, 30, 46, 62, 100) zugegeben wird.

13. Verfahren nach Anspruch 11, wobei das Gewebe-freisetzende Mittel in das zweite Gehäuse (18, 36, 50, 68, 104) zugegeben wird.

14. Verfahren nach Anspruch 11, weiter umfassend das Ausspülen der Zellen, die aus der Gewebeprobe stammen, in einer Vorrichtung zur weiteren Verarbeitung.

15. Verfahren nach Anspruch 11, wobei das Bewegen mindestens eines aus dem ersten und zweiten Gehäuse weiter das Bewegen des ersten Gehäuses (12, 30, 46, 62) relativ zu dem zweiten Gehäuse (18, 36, 50, 68) umfaßt.

16. Verfahren nach Anspruch 11, wobei die Gewebeprobe adipöses Gewebe ist.

17. Verfahren nach Anspruch 11, wobei das Material, enthaltend Zellen, Stammzellen umfaßt.

18. Verfahren nach Anspruch 11, wobei das Gewebe-freisetzende Mittel Collagenase ist.

## Revendications

1. Dispositif (10, 28, 44, 60, 98) destiné à traiter un tissu afin de libérer des cellules à partir du tissu, comprenant :
un premier logement (12, 30, 46, 62, 100) comportant une paroi externe (22, 32, 48, 64, 102) qui présente une forme sélectionnée, le premier logement étant adapté afin de recevoir un échantillon de tissu et la paroi externe comportant une surface externe (22b, 32b, 48b, 64b, 102b) et étant suffisamment poreuse afin de permettre le passage à travers d'une matière comportant des cellules dérivées du tissu ;
un second logement (18, 36, 50, 68, 104) qui renferme au moins sensiblement le premier logement et comporte une paroi externe (19, 37, 51, 71, 105) présentant une forme sélectionnée et une surface interne (19a, 37a, 51a, 71a, 105a) qui est séparée de la surface externe de la paroi externe du premier logement de manière à définir un espace (26, 38, 55) entre elles, l'espace entre la surface externe de la paroi externe du premier logement et la surface interne de la paroi externe du second logement présentant soit une largeur uniforme soit une largeur variable de manière continue ; et
au moins l'un des premier et second logements pouvant se déplacer par rapport à l'autre des premier et second logements afin de faciliter le traitement du tissu dans le premier logement et le passage de matière comportant des cellules dérivées du tissu à travers la paroi externe poreuse du premier logement.

2. Dispositif selon la revendication 1, dans lequel les logements (30, 36, 46, 50, 62, 68) sont adaptés afin de s'assembler sur une base (42, 56, 70).

3. Dispositif selon la revendication 2, dans lequel la base (42, 56, 70) est adaptée de manière à déplacer le premier logement (30, 46, 62) par rapport au second logement (36, 50, 68).

4. Dispositif selon la revendication 3, dans lequel la base (42, 56, 70) est adaptée afin de faire tourner le premier logement (30, 46, 62) par rapport au second logement (36, 50, 68).

5. Dispositif selon la revendication 2, dans lequel la base (70) est adaptée afin de maintenir les premier et second logements (62, 68) sous un angle inférieur à 90° par rapport à une surface sur laquelle la base se repose au cours du traitement.

6. Dispositif selon la revendication 1, dans lequel la paroi externe du second logement (19, 37, 51, 71) est sensiblement rigide.

7. Dispositif selon la revendication 1, dans lequel la paroi externe (22, 32, 48, 64) du premier logement (12, 30, 46, 62) est suffisamment poreuse de manière à permettre le passage à travers d'une matière comportant des cellules présentant un diamètre compris entre 5 environ et 3000 µm environ.

8. Dispositif selon la revendication 1, dans lequel la paroi externe de premier logement (22, 32, 48, 64) comprend, en outre, un panneau formant tamis.

9. Dispositif selon la revendication 1, dans lequel les logements (12, 30, 46, 62, 18, 36, 50, 68) sont adaptés afin de recevoir un agent de libération de tissu en communication avec l'échantillon de tissu.

10. Dispositif selon la revendication 1, comprenant, en outre, un agitateur (106) disposé de manière à pouvoir se déplacer dans le premier logement (100).

11. Procédé de traitement de tissu destiné à libérer des cellules à partir du tissu, comprenant :
a) l'insertion d'un échantillon de tissu contenant des cellules dans un premier logement (12, 30, 46, 62, 100), dans lequel le premier logement comporte une paroi externe (22, 32, 48, 64, 102) présentant une forme sélectionnée et étant suffisamment poreuse de manière à permettre le passage à travers d'une matière comportant des cellules dérivées de l'échantillon de tissu, et dans lequel le premier logement est au moins sensiblement renfermé dans un second logement (18, 36, 50, 68, 104) comportant une paroi externe (19, 37, 51, 71, 105) présentant une forme sélectionnée qui est sensiblement similaire à la forme sélectionnée de la paroi externe du premier logement ou qui varie de manière continue par rapport à la forme sélectionnée de la paroi externe du premier logement ;
b) l'introduction d'agents de libération de tissu dans au moins l'un des logements ; et
c) le déplacement d'au moins l'un des premier et second logements par rapport à l'autre des premier et second logements afin de traiter l'échantillon de tissu et de faire passer la matière comportant des cellules dérivées de l'échantillon de tissu à travers la paroi externe poreuse du premier logement.

12. Procédé selon la revendication 11, dans lequel l'agent de libération de tissu est ajouté au premier logement (12, 30, 46, 62, 100).

13. Procédé selon la revendication 11, dans lequel l'agent de libération de tissu est ajouté au second logement (18, 36, 50, 68, 104).

14. Procédé selon la revendication 11, comprenant, en outre, le passage des cellules dérivées de l'échantillon de tissu vers un dispositif afin d'assurer un traitement complémentaire.

15. Procédé selon la revendication 11, dans lequel le déplacement d'au moins l'un des premier et second logements comprend, en outre, le déplacement du premier logement (12, 30, 46, 62) par rapport au second logement (18, 36, 50, 68).

16. Procédé selon la revendication 11, dans lequel l'échantillon de tissu est un tissu adipeux.

17. Procédé selon la revendication 11, dans lequel la matière comportant des cellules comprend des cellules souches.

18. Procédé selon la revendication 11, dans lequel l'agent de libération de tissu est de la collagénase.
